# EUROPEAN PATENT APPLICATION

(11) **EP 2 055 349 A2**
(43) Date of publication of application: **06.05.2009**
(21) Application number: 08018668.7
(22) Date of filing: 24.10.2008
(51) Int. Cl.: A61N 5/06

(54) **Irradiator with devices for preventing harmful radiation**

(30) Priority: 31.10.2007 DE 102007052108
(71) Applicant: JK-Holding GmbH, 53578 Windhagen (DE)
(72) Inventor: Nanninga, Herman, 1251 AK Laren (NL)
(74) Representative: Koepe, Gerd L.

(57) **Abstract**

The invention relates to an apparatus for irradiating the human body or parts of the human body with ultraviolet radiation, comprising in an arrangement, which allows irradiation of the human body with ultraviolet irradiation in a recumbent, standing, sitting or crouching position, one or more arrangement(s) of radiation sources emitting ultraviolet radiation directed onto a human body in the intended position; devices for positioning the human body to be irradiated in a UV radiation-receiving relationship to the radiation sources; one or more devices for safeguarding the spectral distribution of ultraviolet radiation emitted by UV radiation sources; and devices for electrical or electronic control of the one or more arrangement(s) of radiation sources emitting ultraviolet radiation in the direction of the human body as a function of the data obtained in the one or more above device(s).

## Description

The present invention relates to devices for preventing harmful radiation and further relates to irradiators comprising such devices for preventing harmful radiation.

Irradiators for irradiating the human body or parts thereof (for example the face, the face and upper body, the extremities, the back etc.) have been used for some considerable time for tanning the body or parts thereof or for preparing the body for tanning by natural solar radiation. More recently, the cosmetic, biopositive and medically healing effect of irradiation of the body or parts thereof have become known. In irradiators intended for the above-stated purposes, the human body (whole body irradiators) or parts thereof (partial body irradiators) is exposed to UV radiation, which constitutes an important energy source for many biochemical or biological reactions. To prevent risks to health, UVC radiation is largely eliminated, while the irradiation of UVA radiation and UVB radiation may be achieved and the relative ratio of UVA and UVB radiation may be predetermined in accordance with the objective of the irradiation process.

In the following description and in the claims, the standard phrase "irradiation of the human body with UV radiation" is used, even though in individual cases only part of the human body (for example the face or the face and upper body) is irradiated with UV radiation.

However, owing to their high energy level compared with the other components of natural light, UV rays, whether of natural or artificial origin, are also suited to interacting with the skin. When UV radiation impinges on the skin, a small proportion of the radiation is reflected from the skin surface, while the majority of the UV radiation penetrates into the tissue, where a small proportion is reflected diffusely ("reemitted") as a result of scattering by the molecules of the skin and leaves the body again. By far the greater proportion of the penetrating UV radiation remaining in the tissue is absorbed by the cells directly or after various scattering processes. The depth of penetration of the UV radiation is not the same for all components: While UVB radiation is absorbed for the most part by the epidermis (i.e. a skin layer located between the uppermost stratum corneum and the dermis), over 50% of UVA radiation penetrates into the deeper-lying connective tissue.

The extent to which UV radiation interacts with the cell (for example with a skin cell) depends on whether cellular constituents suitable for absorbing radiation are present, on which the UV radiation impinges and with which a photobiological response is triggered. Such photobiological responses may be responses useful for the body and thus desirably promoted by UV radiation (example: synthesis of vitamin D₃ triggered in the epidermis from just low doses of UVB radiation). However, the photobiological responses may also be disadvantageous responses; examples are molecular changes to DNA (for example mutations), chemical changes to cellular building blocks (proteins, lipids, cell membrane) with subsequent disruption of the regulation of important cellular processes, and sunburn ("erythema formation") on the skin exposed to UV radiation. Particular attention needs to be paid to disadvantageous photobiological responses since they are generally not immediately visible or act in such a way on the human body, even in the case where they soon become visible, that the severity of the damage is not always detectable immediately after UV exposure.

To prevent damage (whether of an immediately detectable or only subsequently manifesting type), technical standards for tanning irradiators and legislative provisions for the operation thereof have recently been enacted, in particular with regard to the professional operation of irradiators ("solariums") in tanning studios. In particular, the most recent recommendations of the radiation protection commission (September 2006: Risks posed to the health of children and young people by UV exposure) and an EU regulation which has very recently become law currently state that irradiators must be operated in such a way that the UV-radiation dose can be limited and a maximum irradiation period can be set.

The previous regulations permitted operation of irradiators for tanning UV radiation, hereinafter known in brief as "tanning apparatuses", at an "erythemally effective" irradiance of approx. 0.6 W/m². The erythemally effective irradiance Eₑᵣ for assessing the biological efficacy of a tanning apparatus is the irradiance determined in the effective area by weighting the measured spectral irradiance with the CIE reference action spectrum for UV erythema. However, in the everyday work of professional operators of tanning studios, substantially higher irradiances were often achieved and the resultant risk of damage accepted.

According to the new regulations, it is only permissible to operate tanning apparatuses at an erythemally effective irradiance of 0.3 W/m², which approximately corresponds to the maximum value of erythemally effective irradiance of the sun to be expected in the tropics at midday at sea level under a cloudless sky. This is intended if not wholly to eliminate the risk of the output of damaging quantities of radiation, at least severely to reduce that risk.

In current commercial tanning apparatuses, which are still generally operated at erythemally effective irradiances of 0.6 W/m², UV tubes are regularly used to generate the emitted, tanning UV radiation. Because of the mixture of phosphors contained in the tubes, when the tubes are operated a radiation spectrum which is characteristic of the mixture of phosphors in the respective tube is emitted. The phosphors in the tubes and thus also the achievable emitted UV spectrum depend on country-specific regulations and also on the preferences of the operator of the tanning apparatuses(private or professional operator).

In particular for professional operation of tanning apparatuses, tubes are available which emit radiation with a relatively high UVB content. These have the disadvantage that people with a lighter (and thus more sensitive) skin type should be exposed to radiation from an accordingly equipped tanning apparatus for only a short time.

On the other hand, tubes are available which emit radiation with a very high UVA content and a small UVB content. These have the advantage that they bring about good "immediate pigmentation" due to melanin delivered to the surface of the skin and there darkened by UVA radiation, so lending the skin a brown colour. A disadvantage, however, is that this pigmentation does not last for long and the longlasting pigmentation, which arises only after a delay due to its association with new pigment production, is only slight. Moreover, when the UVB content emitted in the radiation is low, "solar keratosis", a thickening of the stratum corneum of the skin to provide natural protection against ultraviolet radiation, cannot be built up.

Between tube-emitted radiation with low UVA and high UVB contents on the one hand and radiation with high UVA and low UVB contents on the other hand, numerous spectral variations are feasible; to achieve these, an extremely wide variety of types of tubes emitting UV radiation are available.

Once the (private or professional) operator has decided to equip his/her tanning apparatus with tubes emitting a particular type of UV radiation, the UV spectrum which the tubes of the particular apparatus emit is fixed and can no longer be changed. While, in addition to controlling the radiation dose over time, it is indeed possible to influence irradiance by means of different electrical activation of the tubes used in the tanning apparatus, it is not possible to influence the relative spectral distribution of the radiation (UVA/UVB) relevant for tanning action. Thus, the photobiological action of the tanning apparatus is largely defined by the choice of tubes. The maximum tanning-effective radiation dose is thus determined by means of the irradiation time and the tanning-effective irradiance specific to the UV spectrum of the tubes used in the tanning apparatus.

It was an object of the invention to provide devices with which the output of harmful quantities of radiation to users of irradiators for UV radiation, in particular harmful quantities of tanning UV radiation to users of tanning apparatuses, is prevented, and to provide irradiators for radiation comprising such devices.

It was a further object of the invention to provide devices which make it possible to create the conditions for irradiation of the body of a user with a dose which is reliably below the erythema-producing radiation dose for professional tanning operations irrespective of subjective estimates.

It was a further object of the invention to provide devices with which purposeful manipulation of the output radiation dose of an irradiator by users not experiencing satisfactory tanning is made more difficult, if it cannot be prevented.

It was a further object of the invention to provide irradiators for outputting UV radiation, in particular for outputting tanning UV radiation, including the above-stated devices.

The invention accordingly relates in a first aspect to a device for safeguarding the spectral distribution of ultraviolet radiation emitted by UV radiation sources in apparatuses for irradiating the human body with ultraviolet radiation, comprising means for ensuring non-interchangeable connection of only those radiation sources whose spectral distribution and dose of the emitted UV radiation, thanks to their construction, permit compliance with the limit value for regulated erythemally effective irradiance.

Preferred embodiments of this device are claimed in dependent claims 2 to 5.

In a second aspect, the present invention relates to a device for safeguarding the spectral distribution of ultraviolet radiation emitted by UV radiation sources in apparatuses for irradiating the human body with ultraviolet radiation, comprising means for varying the spectral distribution of ultraviolet radiation emitted by UV radiation sources.

Preferred embodiments of this device are claimed in dependent claims 7 to 9.

In a third aspect, the present invention relates to a device for safeguarding the spectral distribution of ultraviolet radiation emitted by UV radiation sources in apparatuses for irradiating the human body with ultraviolet radiation, comprising means for automatic adjustment of the values of emitted ultraviolet radiation doses of a spectral distribution of UV radiation emitted by UV radiation sources in accordance with the results from a measurement of the skin type and/or skin status of a person to be irradiated.

Preferred embodiments of this device are claimed in dependent claims 11 to 14.

In a fourth aspect, the present invention relates to a device for safeguarding the spectral distribution of ultraviolet radiation emitted by UV radiation sources in apparatuses for irradiating the human body with ultraviolet radiation, comprising means for detecting the UV radiation dose actually emitted over the entire UVA and/or UVB spectral range in the area, and/or over the time, of actual impingement thereof on the irradiated human body (actual data) and means for comparison with predetermined nominal data and optionally means for automatically limiting the UVA and/or UVB radiation dose and/or the irradiation time.

Preferred embodiments of this device are claimed in dependent claims 16 and 17.

The invention finally also relates to an apparatus for irradiating the human body or parts of the human body with ultraviolet radiation, comprising
- in an arrangement which allows irradiation of the human body with ultraviolet radiation in a recumbent, standing, sitting or crouching position,
- one or more arrangement(s) of radiation sources emitting ultraviolet radiation directed onto a human body in the intended position;
- devices for positioning the human body to be irradiated in a UV radiation-receiving relationship to the radiation sources;
- one or more devices for safeguarding the spectral distribution of ultraviolet radiation emitted by UV radiation sources, according to claims 1 to 17; and
- devices for electrical or electronic control of the one or more arrangement(s) of radiation sources emitting ultraviolet radiation in the direction of the human body as a function of the data acquired in the one or more device(s) according to claims 1 to 17.

Preferred embodiments of this apparatus are revealed by dependent claims 19 and 20.

The present invention is explained in greater detail below with reference to the figures, which illustrate preferred but non-limiting embodiments of the invention. In the drawings:
Figure 1 is a graph revealing the shorter pigmentation times (t_{pig}) achieved by low pressure tanning lamps in the body area of the person to be irradiated in a comparison between irradiators according to the present invention (◆) and conventional irradiators (■);
Figure 2 is a graph revealing the shorter pigmentation times (t_{pig}) achieved by face tanning lamps in the face area of the person to be irradiated in a comparison between irradiators according to the present invention (◆) and conventional irradiators (■);
Figure 3 presents all the elements of an irradiator ("sensor tanning apparatus") provided with the devices of the present invention and shows how they interact;
Figure 4 is a flowchart showing the program run before the start of the irradiation process to acquire data about the person to be irradiated; and
Figure 5 is a flowchart showing the program after data has been acquired about the person to be irradiated up to the start of the irradiation process.

The invention will now be explained in greater detail with reference to the preferred embodiments thereof, but is not limited to the preferred embodiments, which are intended primarily to assist in understanding the invention.

The devices provided according to the present invention are not limited in their general embodiment or in their preferred embodiments to implementation with a particular type or a particular make of UV irradiator for irradiating the human body, but rather may be put to general use.

The terms "UV irradiator" or "tanning apparatus" are understood in the following description and in the claims to mean apparatuses with which the body of a user, conventionally of a person, is tanned by means of UV radiation, which at least contains tanning UV radiation even it does not consist solely of tanning UV radiation. This long-known process consists in the irradiation of radiation, preferably UV radiation, still more preferably UV radiation with at least proportions of tanning UV radiation, which comprises UVA and UVB radiation of a per se known wavelength and irradiance composition. The tanning radiation is emitted by suitable radiation sources, for example by suitably pressurised UV tubes or UV radiation emitters (low pressure radiation emitters, high pressure radiation emitters) or UV radiation-emitting LEDs, onto the human body or at least parts thereof. Tanning apparatuses include those apparatuses which serve to irradiate the entire body of a user and those which serve to irradiate parts of the human body (for example face tanning apparatuses or upper body apparatuses), and also those which serve to irradiate the entire body of a user and comprise additional devices (such as for example additional burners and/or tubes and/or LEDs), with which individual parts of the body (e.g. the face, the shoulders, the sides) may additionally be irradiated with UV radiation at the same time as the user's entire body is irradiated. Particularly preferred tanning apparatuses according to the invention are whole body tanning apparatuses, whole body tanning apparatuses being still more preferred which comprise additional devices for targeted irradiation of particular body parts, for example irradiating the face and/or the shoulders.

Tanning apparatuses of the above-described type are known from the prior art and are described therein in detail. Reference may be made, for example, to a detailed description in prior patent application no. DE 10 2005 030 388.9, the content of which relating to the basic structure of a tanning apparatus is incorporated by reference into the present application.

A preferred embodiment is a tanning apparatus conventional in the prior art and provided for tanning the user when recumbent. However, the invention is not limited to such apparatuses, but rather may in principle also be provided for apparatuses in/on which a user is tanned in the sitting or in semi-recumbent or crouching or standing position.

Such generic tanning apparatuses preferably provided for tanning in a recumbent position conventionally consist of a stationary lower part, which, in addition to the UV radiation source part, comprises the electrical units, switching devices and connecting devices needed for operation of the apparatus and optionally also further devices such as for example a cooling system or a ventilation system or devices for multimedia provision, and a mobile upper part. To assist the user to position him/herself on/or in the tanning apparatus, said mobile upper part is at least partially removed from the stationary lower part using suitable devices, for example by tilting or swivelling into an open position about joints or hinges, which generally (but not limitingly) connect one of the longitudinal sides of the lower part movably to one of the longitudinal sides of the upper part, and is returned to its original position once the user has taken up his/her position, whereby a (for example) tunnel-shaped tanning chamber is formed around the user. Alternatively, the upper part may be lifted or swivelled away from the lower part by a separate suspension and may be lowered or swivelled back towards the lower part once the user has taken up his/her position on the lower part. The same process takes place in reverse after completion of the tanning process.

For the user of the tanning apparatus to lie down during the tanning process, the stationary lower part comprises a lying surface which is permeable at least to tanning UV radiation and generally (but not limitingly) largely transparent.

This consists of a material permeable at least to tanning UV radiation. This may be any material known to a person skilled in the art which is largely transparent to UV radiation. This material not only has the necessary transparency for the UV fractions of the radiation, but instead also displays further advantageous characteristics, such as for example sufficient stability and flexibility to bear the person to be irradiated and excellent resistance against the action of cleaning and disinfecting substances, which have to be used after the tanning process. An acrylic polymer is advantageously used.

Below the lying surface there is arranged, preferably in the longitudinal direction of the tanning apparatus, at least one set of radiation sources for UV radiation, in particular for tanning UV radiation, particularly preferably lamp tubes emitting tanning UV radiation, but conceivably also UV radiation-emitting LEDs. The radiation is directed, as prescribed, in the direction of the upper part of the tanning apparatus and thus of the user recumbent on the lying surface of the tanning apparatus, or at least at a part of his/her body or a plurality of parts of his/her body. Optionally, it is possible, in addition to the stated lamp tubes, also be arrange one or more further radiation sources in the lower part of the tanning apparatus, these ensuring targeted tanning of particular areas of the user's body.

The upper part of the tanning apparatus connected as described above to the stationary lower part and cooperating therewith comprises a protective surface for the user which is substantially permeable at least to tanning UV radiation, which protective surface is arranged between the user and the electrically operated units of the upper part. This protective surface may consist of any desired material known to a person skilled in the art on the basis of his/her specialist knowledge which is substantially permeable at least to tanning UV radiation and is sufficiently stable. Preferably, an acrylic polymer is used for the protective surface, as is also used for the lying surface of the lower part. In a preferred embodiment, the protective surface is shaped in such a way that a (for example, but not limitingly) tunnel-shaped tanning chamber is formed over the user of the tanning apparatus recumbent under the protective surface, the highest part of the tunnel preferably lying approximately in the middle of the upper part.

Above the protective surface (preferably forming a tunnel-shaped tanning chamber) there is arranged at least one set of radiation sources for UV radiation, in particular for tanning UV radiation, preferably arranged in the longitudinal direction of the tanning apparatus. The arrangement of the UV radiation sources in the upper part of the tanning apparatus takes account of the preferred (from the standpoint of the UV radiation source) convex shape of the protective surface. The UV radiation sources are in most cases tanning UV radiation-emitting lamp tubes, but may also be other UV radiation sources familiar to a person skilled in the art, such as for example UV radiation-emitting LEDs. The radiation is directed, as prescribed, in the direction of the lower part of the tanning apparatus and thus of the user recumbent on the lying surface of the tanning apparatus, or at least at a part of his/her body or a plurality of parts of his/her body. In addition to the stated lamp tubes, it may be possible for one or more further radiation sources also to be arranged in the upper part of the tanning apparatus, these being directed specifically at particular parts of the user's body, such as for example at the user's face and ensuring targeted tanning.

Tanning apparatuses for tanning a user when standing or sitting are constructed in accordance with the user's other than recumbent position, but substantially comprise the same components essential for irradiation with UV radiation as the above-described apparatus for tanning in the recumbent position, which is preferred according to the invention.

Such a per se known tanning apparatus optionally comprising further devices useful or advisable for irradiation of a user's body will now be further described in connection with the present invention.

To prevent the occurrence of harmful radiation, various devices for safeguarding the spectral distribution of ultraviolet radiation emitting by the UV radiation sources (e.g. UV lamp tubes, UV burners) are used in conjunction with the UV irradiators or tanning apparatuses described in general terms above when irradiating the human body, for example the body of a user of such an irradiation or tanning apparatus. According to the invention, various devices are used, which all have the same objective, namely preventing the occurrence of doses of harmful ultraviolet radiation in tanning apparatuses which are injurious to health and thus dangerous, whether as a result of unintentional incorrect operation and malfunctioning of the tanning apparatus or as a result of deliberate manipulation of the tanning apparatus by a foolish user unconcerned about his/her health.

According to one aspect of the invention, such a device for safeguarding the spectral distribution of ultraviolet radiation emitted by UV radiation sources comprises means for ensuring non-interchangeable connection of only those radiation sources whose spectral distribution and dose of the emitted UV radiation, thanks to their construction, permit compliance with the limit value for regulated erythemally effective irradiance. Such means are intended to ensure that, without the risk of unintentional exchange or deliberate, risk-entailing manipulation by incorrect installation of radiation sources such as for example UV radiation lamp tubes, only those UV radiation sources are installed in tanning apparatuses which output a UV radiation spectrum which is safe for the tanning process. The installation of lamp tubes outputting an admissible spectrum of UV radiation is conventionally design-related for professional tanning apparatuses and is subject to safety testing in line with technical specifications. The circumvention of such specifications, whether through negligence or whether through deliberate circumvention of safety-relevant regulations, is frequently observed but is rendered impossible by the present devices. Specifically, only those UV radiation sources can be installed which comply with the specifications relating to radiation safety; compliance with the limit values of regulated erythemally effective irradiance is thus possible when using such radiation sources.

The above-described means may be provided in an irradiator/tanning apparatus alone or together with one or more of the other means described below in the present description.

In preferred embodiments of the invention, the UV radiation sources are radiation sources for emitting tanning UV radiation, particularly preferably in apparatuses for irradiating the human body in tanning apparatuses according to the above definition. The UV radiation sources are also preferably radiation sources for emitting tanning UVA and/or tanning UVB radiation and/or the apparatuses for irradiating the human body with ultraviolet radiation are preferably tanning apparatuses with tanning UVA radiation- and/or tanning UVB radiation-emitting lamp tubes.

In accordance with a further preferred embodiment of the invention, the means for ensuring non-interchangeable connection of radiation sources are mechanical safeguarding means. This means that the safeguarding function is achieved in that non-interchangeability is brought about mechanically. Such mechanical means for ensuring non-interchangeable connection of radiation sources may also preferably be safeguarding means associated with the shape of the connection, i.e. they function in such a way that the geometric shape of the connection of the UV radiation source is crucial with regard to bringing the UV radiation source into operation in the tanning apparatus and ensuring that geometric shapes which do not fit cannot be brought into operation. For example, lamp tubes with a round electrical connection cannot be connected to a square socket (and vice versa). By specifying the socket, specialised lamp tubes may be provided for exclusive use, while lamp tubes with other sockets are discriminated against.

In a further, likewise preferred embodiment of the invention, the means for ensuring non-interchangeable connection of radiation sources are safeguarding means associated with tube length. This means that to safeguard the desired spectral distribution of the UV radiation of a tanning apparatus, the lamp tubes which cover different areas of the UV radiation spectrum have different lengths and the spectral UV distribution between UVA and UVB radiation allocated to an apparatus is fixed by specifying installation spaces of lengths corresponding to the various lengths of the various tubes. In this way, it is impossible to equip one side of a tanning apparatus solely with UVA lamp tubes or solely with UVB tubes.

In accordance with a further preferred embodiment of the invention, the means for ensuring non-interchangeable connection of UV radiation sources are electrical and/or electronic safeguarding means. Examples of these are only too familiar to a person skilled in the art. Preference is further given to those devices in which the means for ensuring non-interchangeable connection of radiation sources are also preferably means for outputting and/or detecting an electronic code of the radiation sources used. Such devices for outputting or detecting an electronic code on parts of the apparatus which are designed to be connected together electrically are very well known to a person skilled in the art in this technical field and do not therefore need to be explained in detail here.

According to a further preferred embodiment of the invention, combinations of the two above-stated safeguarding means are also feasible, i.e. devices in which the means for ensuring non-interchangeable connection of radiation sources comprise a combination of mechanical and electr(on)ic safeguarding means. Such combinations are particularly preferred, since they further increase security against the output of harmful radiation and thus further reduce the risk to the health of the person to be irradiated.

According to a further aspect of the present invention, a device for safeguarding the spectral distribution of ultraviolet radiation emitted by UV radiation sources in apparatuses for irradiating the human body with ultraviolet radiation comprises means for varying the spectral distribution of ultraviolet radiation emitted by UV radiation sources. In this way, the emitted UV radiation may advantageously be controlled dynamically or by switching. These means may be provided in an irradiator/tanning apparatus alone or together with one or more of the other means described below in the present description.

In one embodiment of the invention, a prerequisite for this may be that two complementary UV spectra of different types from different lamp tubes are present. The tubes which differ with regard to their spectral distribution of the emitted radiation may be used in the context of selection of the relative number of the different types of lamp tubes and/or in the context of separate electrical activation and/or switching on or off in order purposefully to influence the photobiological characteristics of the tanning apparatus.

In a preferred embodiment of the invention, the means for varying the spectral distribution of ultraviolet radiation emitted by UV radiation sources are means for varying how the UV irradiator is equipped with radiation sources with primarily UVA radiation emission and with radiation sources with primarily UVB radiation emission, so establishing a variable ratio of UVA radiation/UVB radiation. In other words: to equip a tanning apparatus with UV lamp tubes, tubes which primarily emit UVA radiation and tubes which primarily emit UVB radiation are used next to one another. This may proceed as a function of various parameters, one of which, for example, is the distance between the person to be irradiated and the set of emitting UV lamp tubes, which, for example in the case of a whole body tanning apparatus for use in the recumbent position, is greater by design in the upper part of the tanning apparatus than in the lower part.

In a further preferred embodiment of the invention, the means for varying the spectral distribution of ultraviolet radiation emitted by UV radiation sources are means for adjusting the emitted quantity of UV radiation from the UVA/UVB spectral ranges. This means that the relative ratio of the number of lamp tubes primarily emitting UVA radiation to the number of lamp tubes primarily emitting UVB radiation in the context of comparable emissions of radiation or the quantity of radiation emitted by different numbers of UV lamp tubes is set to a predetermined ratio and the risk of overdosing with one radiation component, with its attendant risk to health, is eliminated.

It is also preferable according to the invention for the means for varying the spectral distribution of ultraviolet radiation emitted by UV radiation sources to be means for adjusting the quantity of emitted UV radiation from the UVA/UVB spectral ranges to a value in the range from 0.5:1 to 5:1. This may proceed for example by means of the number of lamp tubes primarily emitting the respective radiation. Merely by way of example, the number of lamp tubes emitting primarily UVA radiation in the lower part of a tanning apparatus, where the tanning distance between emitting lamp tubes and the person to be irradiated is small, is equal to the number of tubes emitting primarily UVB radiation. From a purely practical standpoint, the tubes are therefore fitted alternately. In contrast, in the upper part of a tanning apparatus, where the distance from the person to be irradiated is greater, a number of tubes emitting primarily UVB radiation are fitted, in addition to 3 to 4 tubes emitting primarily UVA radiation.

According to the invention, it is also preferred for the means for adjusting the quantity of emitted UV radiation from the UVA/UVB spectral ranges to comprise control of the number of UV radiation sources primarily emitting UVA radiation relative to the number of UV radiation sources primarily emitting UVB radiation. Alternatively, the means for adjusting the quantity of emitted UV radiation from the UVA/UVB spectral ranges comprises control of electrical activation of the UV radiation sources primarily emitting UVA radiation relative to the electrical activation of the UV radiation sources primarily emitting UVB radiation. This may be achieved, in further preferred manner, in that control of the electrical activation of given UV radiation sources takes place by switching on and off radiation sources primarily emitting a given UV radiation (for example UV lamp tubes primarily emitting UVB radiation), or in that control of the electrical activation takes place by dimming radiation sources primarily emitting a given UV radiation (for example dimming of the lamp tubes primarily emitting UVB radiation).

Instead of the number of tubes operated under identical conditions, it is thus also possible to vary the time over which the various tubes emit their respective UV spectrum. Time control ensures in comparable manner a relative proportion of the quantity of radiation in the above-stated range of, particularly advantageously, 0.5:1 to 5:1. In the same way, according to the invention, control of the various types of tubes is possible via the quantity of energy which leads to varying intensities of emission by the respective tube type. This means that, in a practical example, the UV lamp tubes primarily emitting UVA radiation may be operated not only with normal ballast electronics (160/180/200 watts), but also with an electronic, non-dimmable ballast. The lamp tubes primarily emitting UVB radiation may be switched on or dimmed.

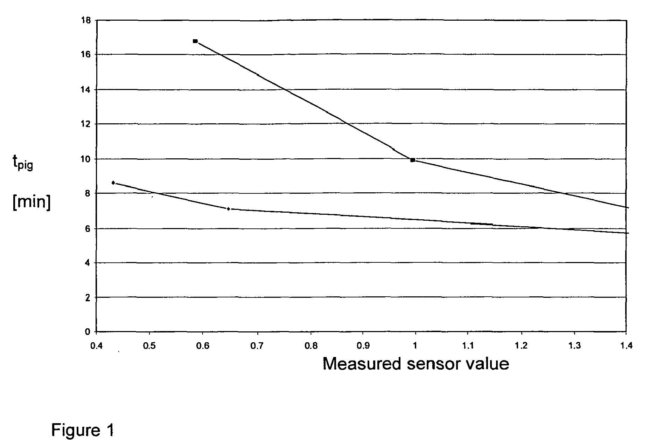

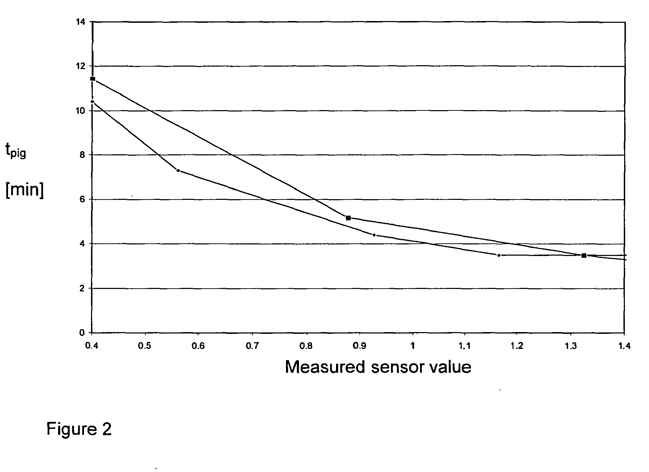

In practice, the variability of the spectrum emitted onto the body of the person to be irradiated may result in a markedly shorter pigmentation time (t_{pig}) than in the prior art, as is apparent from Figures 1 and 2. Skin pigmentation measured with a pigmentation status sensor is plotted in Figure 1, for a low pressure tanning lamp, as may be found in the body area of the tanning apparatus, against the time necessary to achieve this pigmentation: The curve marked "■" shows the prior art without variable tanning spectrum, while the curve marked "◆" shows the variable spectral adjustment according to this aspect of the invention. It is apparent that the pigmentation time according to the invention is markedly shorter, which is associated with an absolute and relative (due to the dimmed or switched-off lamp arrangement) energy saving in addition to preventing the risk of an overdose of UV radiation.

In Figure 2, the arrangement is comparable to that of Figure 1 but plotted here for a face tanning lamp. Here too, shorter pigmentation times t_{pig} are achieved for the skin pigmentation values (measured by a pigmentation sensor).

According to a further aspect of the invention, a device for safeguarding the spectral distribution of ultraviolet radiation emitted by UV radiation sources in apparatuses for irradiating the human body with ultraviolet radiation comprises means for automatic adjustment of the values of emitted ultraviolet radiation doses of a spectral distribution of UV radiation emitted by UV radiation sources in accordance with the results from measurement of the skin type and/or skin status of a person to be irradiated.

These means may be provided in an irradiator/tanning apparatus alone or together with one or more of the other means described below in the present description.

In accordance with the present invention, such means are used for automatic adjustment of emitted values of ultraviolet radiation doses of a spectral distribution of UV radiation emitted by UV radiation sources. This should be understood to mean that adjustment of a given spectrum of the UV radiation emitted and acting on the body of the person to be irradiated is not effected manually (for example by the user) and not as decided by a person (in professional tanning studios for example by the studio staff), but rather automatically after measurement of the skin type by communicating the measurement result to the apparatus and adjusting the corresponding control of the UV radiation-emitting lamp tubes.

In a preferred embodiment of the invention, the measurement or measurements of the skin type and/or skin status of the person to be irradiated is/are performed optically. In known manner, a suitable measuring arrangement is positioned on different points of the skin of the person to be irradiated (in this context any desired measuring arrangement may be used, and purely by way of example, without in this way limiting the invention, reference is made to the measuring arrangement of the applicant for the present application in document DE-A 103 38 004). Skin type and/or skin status, in particular the pigmentation or tanning status of the skin, is determined in a further preferred manner by a light reflection measurement, more preferably by a light reflection measurement using light of at least two different wavelengths. In a very particularly preferred manner, this measurement takes place by means of light from at least two LEDs with different light emission. In this way, so it has been found, it is possible to establish highly reliably a picture of the skin type of a person and, as is likewise reliably possible, a picture of the current skin status, at least as regards pigmentation status/tanning status. This method is based on the idea that people with light skin, which is conventionally more highly sensitive to UV radiation, and/or with skin which is unused to UV radiation should receive only a relatively small dose of tanning UV radiation compared with people at the other end of the scale with dark skin, which may have already been prepigmented by previous solar irradiation, for whom the risk of damage (for example the occurrence of sunburn (erythema)) as a result of excessive doses of UV radiation is substantially lower.

At least two points on the surface of the skin of the person to be irradiated should be recorded instrumentally in the manner described for skin type and/or skin status/tanning status, for which purpose the face or the arms (i.e. body parts coming more frequently into contact with UV radiation) and a point on the stomach or back (i.e. a body part generally coming less frequently into contact with UV radiation) are more preferably used.

As also in the case described above, a number of irradiation parameters may be adjusted as a result of measurement of skin type and/or skin status, in order to safeguard against excessive doses of UV radiation: In preferred embodiments, the means for automatic adjustment of the values of UV radiation doses emitted onto the human body lead to the automatic adjustment of maximum values for such emitted radiation doses, i.e. to values for the radiation quantity emitted by the UV radiation sources which must not be exceeded. As an alternative thereto, or together therewith, values for emitted radiation doses per predetermined unit time may be automatically set, which then likewise leads by means of summation over the selected or predetermined total period to maximum values for the total dose, but allows time variation. As an alternative thereto, or together therewith, automatic adjustment of the maximum irradiation time may be undertaken even when the time-related UV radiation dose has been predetermined; this makes sense for example in the case of first irradiation exposure, during which it is desired, slowly and with a low dose, to approach practicable irradiation times which preferably amount of 20 minutes. It goes without saying that any other desired (otherwise freely variable) irradiation parameters may be automatically set, without the invention thereby being limited. The sole criterion is the dependency of automatic adjustment on the result of the instrumental determination of skin type and/or skin status, in particular skin tanning status.

In an alternative, simplified measuring arrangement, which is also less precise, however, and thus entails the risk of mismeasurement, the individual skin type may also be measured in a tuned multi-stage circuit which leads to adjustment of the apparatus within a relatively coarse framework for the individual skin type. Optionally, the coarser adjustment, if initiated, may be followed by fine measurement using the sensor means according to the invention and subsequent automatic adjustment.

In practical operation, a fixed irradiation time is preferably provided as a result of the sensor measurement, which is more preferably for example 20 minutes. The lamp tubes primarily emitting UVA radiation are preferably operated with maximum possible activation over the entire tanning period. The automatic adjustment undertaken as a result of the sensor measurement is intended to ensure that sunburn/erythema cannot occur. The apparatus control system (generally provided in software form) has to control/switch the lamp tubes in such a way as a result of the sensor measurement input that the target time is complied with and the lamp tubes, for example the UVB radiators, otherwise emitting too much radiation are dimmed or switched off on a timed basis. Dimming of UVA radiators is conceivable for people with extremely sensitive skin, though it would seldom arise; switching off of UVA radiators is to be expected, but rather these are normally operated at as far as possible maximum power, as stated above, for more or less the entire irradiation time.

In a particularly preferred embodiment of the invention, in the event of first irradiation of a person to be irradiated, the skin type is adjusted by means of the automated measurement to the highest skin sensitivity setting, in order in any case to prevent damage and erythema formation. Alternatively, in the case of a first tanning session, a fixed radiation dose may be preset, for example preferably a maximum erythemally effective radiation dose of 100 J/m².

In principle, of course, operation of a tanning apparatus without the use of a sensor is technically conceivable, but is regarded as a less preferred embodiment of the invention for reasons of safeguarding against harmful UV radiation doses.

According to the invention, it is further preferred for the means for automatic adjustment of values for emitted ultraviolet radiation doses, as far in particular as long-term ongoing irradiation with UV radiation is concerned, to allow the inclusion of personal tanning data relating to a person to be irradiated. These may be used for example in order to determine maximum UV radiation doses over extended periods of time or to verify earlier settings of the tanning apparatus used by an individual.

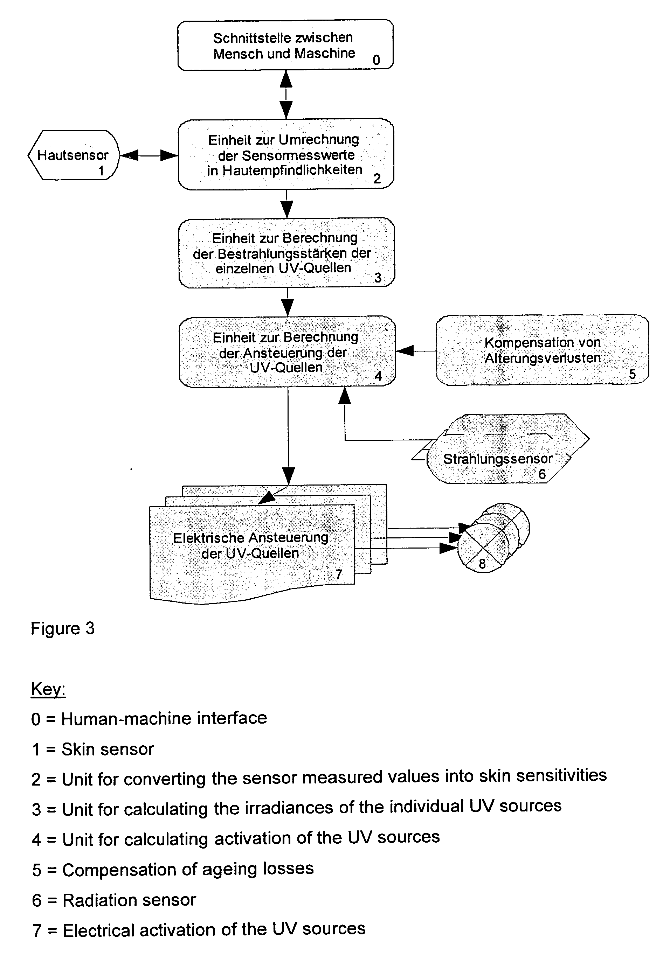

Figure 3 shows a list of all the elements of a tanning apparatus comprising the means according to the above description and the interaction thereof.

An "element" here means a self-contained task or unit. These are embodied in apparatus components, it preferably being possible to combine various elements in one component. Elements 3 to 6 are implemented in current generation tanning apparatuses for example within the control electronics as a component of the software.

Not all the elements are absolutely essential and they do not therefore have to be implemented in all tanning apparatuses; sometimes they are only implemented in part or not for all UV radiation sources.

### - Human-machine interface

This interface serves to give the user instructions on handling the system. This may take place acoustically or via a display, in text or symbol form.

In an extended configuration stage, extra information may be acquired from the user by the system via an additional input option. This configuration stage is not absolutely essential.

### - Skin sensor

The skin sensor has the task of collecting physical measured values about the skin. In a preferred sensor, the reflection values of variously coloured light sources are determined to this end.

In principle, other measurement methods are also conceivable, provided they supply sufficient information for subsequent calculation of skin sensitivity.

The measurement sequence has optionally, and in accordance with the structure of the tanning apparatus, to determine the characteristics of the skin at different points on the body. In preferred tanning apparatuses, a distinction is drawn between the face area and the body area.

### - Element for converting the measured sensor values into skin sensitivities

In this element, a value is calculated for the "sensitivity" of the skin to UV irradiation. The basis therefor is the measured values from the sensor and optionally the additional information obtained from the customer via the human/machine interface (see above).

Depending on the configuration of the apparatus, one value per irradiation zone is here calculated.

The calculated values are then converted into a radiation dose per zone.

### - Element for calculating irradiance

In this element, the calculated radiation dose is shared out per zone between the individual irradiation sources and converted into irradiances. Depending on the configuration of the tanning apparatus or of the UV radiation sources, power adaptation or partial or full switching off of individual sources may be determined to achieve the predetermined dose.

In this element, partial doses may also be redistributed within certain limits. In this way, the tanning results may be influenced with the same total dose. In preferred apparatuses, redistribution between high pressure and low pressure lamps is for example feasible in the face area.

### - Element for calculating activation of the radiation sources

This element calculates activation for achieving the desired irradiances of each UV source.

To this end, the algorithm must know the dependencies between activation and output power of each source. This may take place in the form of constant values, which are stored in software. Likewise, it is possible by means of an additional element to make ageing-dependent information known to the algorithm.

This element may also be designed as a feedback control circuit, in which up-to-date information about irradiance is provided to the algorithm via a sensor element during running time. Said algorithm may then optionally modify activation.

### - Compensation of ageing losses

This optional element serves to process information about the ageing of individual UV sources and to determine changes to activation and output power.

The determined dependencies are used to calculate activation.
In our current apparatuses, the ageing of the low pressure lamps in the body area is compensated by increasing basic activation on the basis of a known ageing curve as a function of the operating hours of the lamps.

### - Radiation sensor

This optional element enables the configuration of a feedback control circuit. It serves to determine physical variables relevant to the assessment of irradiance.

Both direct light or UV values and auxiliary variables such as electrical power or heat development may in this case be determined.

The element calculates from these measured values the actual radiant power of individual UV sources and communicates the values to the activating element.

In preferred embodiments, the supply voltage of the tanning apparatus is measured and integrated into the feedback control circuit.

### - Electrical activation

This element generally consists of a plurality of components and provides electrical activation of the UV sources. The different characteristics of starting and continuous running behaviour have here to be taken into account.

### - UV sources

The UV sources of the tanning apparatus are divided, depending on the configuration of the apparatus, into different zones such as for example body and face. Each zone may in this respect contain a plurality of and also different UV sources.

According to a further aspect of the present invention, devices for safeguarding the spectral distribution of ultraviolet radiation emitted by UV radiation sources in apparatuses for irradiating the human body with ultraviolet radiation comprise means for detecting the UV radiation dose actually emitted over the entire UVA and/or UVB spectral range in the area, and/or over the time, of actual impingement thereof on the irradiated human body (actual data) and means for comparison with predetermined nominal data and optionally means for automatically limiting the UVA and/or UVB radiation dose and/or the irradiation time.

These means may be provided in an irradiator/tanning apparatus alone or together with one or more of the other means described below in the present description.

According to the invention, the means for detecting the UV radiation dose actually emitted over the entire UV spectral range (UVA and UVB radiation) exposed to emission are fitted in an area which is covered by the emitted radiation, in practice (but limitingly) in the vicinity of the human body exposed to irradiation. Detection of the radiation which in practice represents the actual value of the emitted UV radiation and may likewise be used according to the invention for adjusting a safety standard, may detect the emitted radiation over the entire UVA range (i.e. a wavelength range of 320 to 400 nm) or over the entire UVB range (i.e. a wavelength range of 280 to 320 nm) or over both ranges (UVA + UVB; i.e. a wavelength range of substantially between 280 and 400 nm). In the same way, the means detects the emitted UV radiation over the entire emission period, i.e. the period during which the human body is exposed to the UV radiation emitted by the UV lamp tubes. A comparison possible at any time after technical set-up of the means with predetermined nominal values, for example with maximum values for the UV radiation received, may lead to subsequent automatic limitation of the UVA and/or UVB radiation dose and/or the irradiation period, in order to prevent the user's body from receiving a harmful dose of UV radiation.

In a preferred embodiment, the means are means which enable summary detection of the actual UV radiation dose by means of individual UV radiation ranges or over the entire range of UVA and UVB radiation.

Further preferred embodiments of the invention relate to means in which a sensor or a plurality of sensors are arranged in a plurality of areas, in which the emitted UV radiation has impinged on the body of the person to be irradiated.

Preference is also given to an embodiment of the invention in which one or more sensors are arranged for detecting the UV radiation dose actually emitted over the entire UVA range of the spectrum and/or over the entire UVB range of the spectrum. These may, in each case when predetermined values are approached or indeed exceeded, alone or together initiate automatic limiting of the UVA and/or UVB radiation dose.

In a still more preferred embodiment of the invention, one or more sensors are provided as the means for detecting the UV radiation dose actually emitted over the entire UVA and/or UVB range of the spectrum, which sensors form a measuring system for determining UV irradiance calibrated to the specific UV spectrum of the UV radiation sources used. This has the advantage of a complete monitoring system always taking account of the reference systems and contributes in a particularly advantageous manner to the prevention of overdoses of one or more components of UV radiation.

In an alternative embodiment, the sensor or sensors is/are a device for the direct measurement of UV irradiance.

The invention also relates to an apparatus for irradiating the human body or parts of the human body with ultraviolet radiation. This apparatus may comprise the components known in the prior art.

Preferably, such an apparatus for irradiating the human body or parts of the human body with ultraviolet radiation comprises
- in an arrangement which allows irradiation of the human body with ultraviolet radiation in a recumbent, standing, sitting or crouching position,
- one or more arrangement(s) of radiation sources emitting ultraviolet radiation directed onto a human body in the intended position;
- devices for positioning the human body to be irradiated in a UV radiation-receiving relationship to the radiation sources;
- one or more devices for safeguarding the spectral distribution of ultraviolet radiation emitted by UV radiation sources according to the above detailed description; and
- devices for electrical or electronic control of the one or more arrangement(s) of radiation sources emitting ultraviolet radiation in the direction of the human body as a function of the data acquired in the one or more device(s) according to the above detailed description.

All the features of the devices described in detail above will also be applied mutatis mutandis by a person skilled in the art in accordance with the above-stated information to the UV irradiator according to the invention.

According to preferred embodiments of the invention, the UV irradiator is preferably a tanning apparatus for tanning in the recumbent, standing or sitting position, is preferably a tanning apparatus for tanning in the recumbent position and is more preferably a whole body tanning apparatus for tanning in the recumbent position.

In the case of a tanning apparatus for tanning in the recumbent position, which is particularly preferred according to the invention, still more preferably a whole body tanning apparatus for tanning in the recumbent position, said apparatus comprises the following components, for example:
- a stationary lower part with head end and foot end and two longitudinal sides, containing electrical units, switching devices and connecting devices; and
- an upper part with head end and foot end and two longitudinal sides, either fitted to one of the longitudinal sides of the lower part via hinges or joints, tiltable or swivellable by means of the hinges or joints away from the lower part and towards the lower part to form a tanning chamber or liftable away from the lower part and lowerable towards the lower part to form a tanning chamber by means of a separate suspension; wherein
- the lower part comprises a lying surface for the user which is permeable at least to tanning UV radiation and at least one set of lamps arranged beneath the lying surface in the longitudinal direction of the tanning apparatus and optionally also further lamps for outputting radiation with at least a proportion of tanning UV radiation towards the upper part and/or at least a part of the user's body, in direct or indirect connection with the electrical units, switching devices and/or connecting devices; and
- the upper part comprises a protective surface for the user which is permeable at least to tanning UV radiation and at least one set of lamps arranged above the protective surface in the longitudinal direction of the tanning apparatus and optionally also further lamps for outputting radiation with at least a proportion of tanning UV radiation towards the lower part and/or at least a part of the user's body; and further comprises according to the invention
- one or more devices for safeguarding the spectral distribution of ultraviolet radiation emitted by UV radiation sources according to the above detailed description, which a person skilled in the art may straightforwardly apply mutatis mutandis to the UV irradiator on the basis of the above-stated teaching.

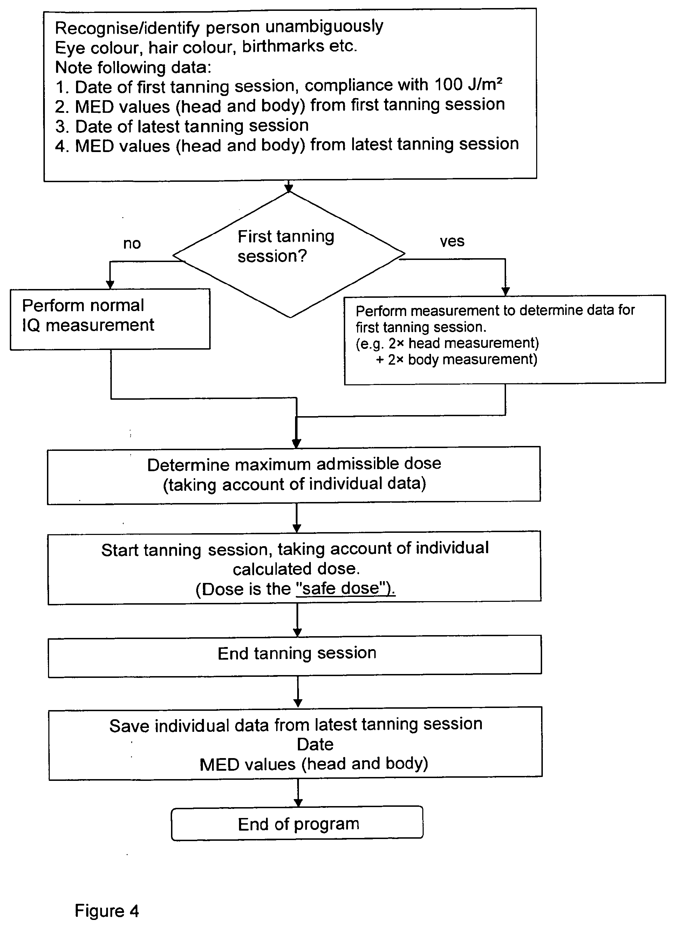

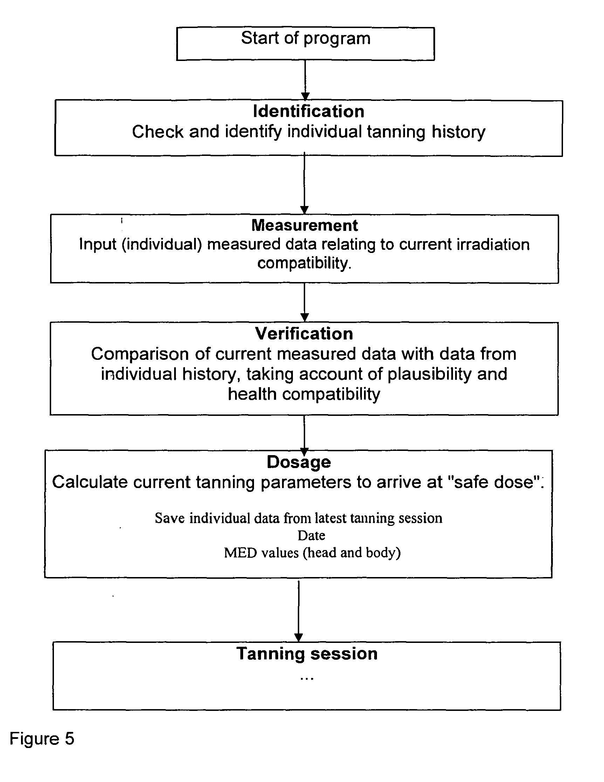

Examples of the procedure for safeguarding the spectral distribution of ultraviolet radiation emitted by UV radiation sources in apparatuses for irradiating the human body are revealed by the two flowcharts in Figures 4 and 5, which do not require any further detail description since they precisely indicate the necessary steps to a person skilled in the art.

## Claims

1. A device for safeguarding the spectral distribution of ultraviolet radiation emitted by UV radiation sources in apparatuses for irradiating the human body with ultraviolet radiation, comprising means for ensuring non-interchangeable connection of only those radiation sources whose spectral distribution and dose of the emitted UV radiation, thanks to their construction, permit compliance with the limit value for regulated erythemally effective irradiance.

2. A device according to claim 1, in which the UV radiation sources are radiation sources for emitting tanning ultraviolet radiation and/or in which the apparatuses for irradiating the human body with ultraviolet radiation are tanning apparatuses, preferably in which the UV radiation sources are radiation sources for emitting tanning UVA and/or tanning UVB radiation and/or in which the apparatuses for irradiating the human body with ultraviolet radiation are tanning apparatuses with lamp tubes emitting tanning UVA radiation and/or tanning UVB radiation.

3. A device according to claim 1 or claim 2, in which the means for ensuring non-interchangeable connection of radiation sources are mechanical safeguarding means, preferably in which the means for ensuring non-interchangeable connection of radiation sources are safeguarding means associated with the shape of the connection or in which the means for ensuring non-interchangeable connection of radiation sources are safeguarding means associated with tube length.

4. A device according to claim 1 or claim 2, in which the means for ensuring non-interchangeable connection of radiation sources are electrical and/or electronic safeguarding means, preferably in which the means for ensuring non-interchangeable connection of radiation sources are means for outputting and/or detecting an electronic code of the radiation sources used.

5. A device according to any one of claims 1 to 4, in which the means for ensuring non-interchangeable connection of radiation sources are a combination of mechanical and electr(on)ic safeguarding means.

6. A device for safeguarding the spectral distribution of ultraviolet radiation emitted by UV radiation sources in apparatuses for irradiating the human body with ultraviolet radiation, comprising means for varying the spectral distribution of ultraviolet radiation emitted by UV radiation sources.

7. A device according to claim 6, in which the means for varying the spectral distribution of ultraviolet radiation emitted by UV radiation sources are means for varying how the UV irradiator is equipped with radiation sources with primarily UVA radiation emission and with radiation sources with primarily UVB radiation emission, so establishing a variable ratio of UVA radiation/UVB radiation, preferably in which the means for varying the spectral distribution of ultraviolet radiation emitted by UV radiation sources are means for adjusting the quantity of emitted UV radiation from the UVA/UVB spectral ranges, more preferably in which the means for varying the spectral distribution of ultraviolet radiation emitted by UV radiation sources are means for adjusting the quantity of emitted UV radiation from the UVA/UVB ranges to a value in the range of 0.5:1 to 5:1.

8. A device according to claim 6 or claim 7, in which the means for adjusting the quantity of emitted UV radiation from the UVA/UVB spectral ranges comprises control of the number of UV radiation sources primarily emitting UVA radiation relative to the number UV radiation sources primarily emitting UVB radiation or in which the means for adjusting the quantity of emitted UV radiation from the UVA/UVB spectral ranges comprises control of electrical activation of the UV radiation sources primarily emitting UVA radiation relative to the electrical activation of the UV radiation sources primarily emitting UVB radiation.

9. A device according to claim 8, in which control of the electrical activation takes place by switching on and off radiation sources primarily emitting a given UV radiation, or in which control of the electrical activation takes place by dimming radiation sources primarily emitting a given UV radiation.

10. A device for safeguarding the spectral distribution of ultraviolet radiation emitted by UV radiation sources in apparatuses for irradiating the human body with ultraviolet radiation, comprising means for automatic adjustment of the values of emitted ultraviolet radiation doses of a spectral distribution of UV radiation emitted by UV radiation sources in accordance with the results from a measurement of the skin type and/or skin status of a person to be irradiated.

11. A device according to claim 10, in which the means for automatic adjustment of values for emitted ultraviolet radiation doses comprise means for automatic adjustment of maximum values for emitted ultraviolet radiation doses, means for adjusting values for emitted ultraviolet radiation doses per predetermined unit time, means for adjusting values for maximum irradiation time with a predetermined, time-related ultraviolet radiation dose and/or means for adjusting other irradiation-relevant parameters.

12. A device according to claim 10 or claim 11, in which the measurement(s) of the skin type and/or of the skin status of a person to be irradiated proceed optically, preferably in which the measurement(s) of the skin type and/or of the skin status of a person to be irradiated proceed by means of light measurement using light of at least two different wavelengths, more preferably in which the measurement(s) of the skin type and/or of the skin status of a person to be irradiated proceed by means of light from at least two LEDs with different emissions.

13. A device according to claim 10 or claim 11, in which the means for automatic adjustment of values for emitted ultraviolet radiation are set, in the case of people undergoing ultraviolet radiation for the first time in an apparatus for irradiating the human body with ultraviolet radiation, to a maximum value for the erythemally effective radiation dose of 100 J/m².

14. A device according to any one of claims 10 to 13, in which the means for automatic adjustment of values for emitted ultraviolet radiation doses allow the inclusion of personal tanning data relating to a person to be irradiated.

15. A device for safeguarding the spectral distribution of ultraviolet radiation emitted by UV radiation sources in apparatuses for irradiating the human body with ultraviolet radiation, comprising means for detecting the UV radiation dose actually emitted over the entire UVA and/or UVB spectral range in the area, and/or over the time, of actual impingement thereof on the irradiated human body (actual data) and means for comparison with predetermined nominal data and optionally means for automatically limiting the UVA and/or UVB radiation dose and/or the irradiation time.

16. A device according to claim 15, comprising means for summary detection of the radiation dose actually emitted by all the UV radiation sources over the entire UVA and UVB spectral range.

17. A device according to claim 15 and claim 16, comprising as means for detecting the UV radiation dose actually emitted over the entire UVA and/or UVB spectral range one or more UV sensor(s), preferably comprising as means for detecting the UV radiation dose actually emitted over the entire UVA and/or UVB spectral range a measuring system for determining irradiance calibrated to the specific UV spectrum of the UV radiation sources used or a device for direct measurement of UV irradiance.

18. An apparatus for irradiating the human body or parts of the human body with ultraviolet radiation, comprising
- in an arrangement which allows irradiation of the human body with ultraviolet radiation in a recumbent, standing, sitting or crouching position,
- one or more arrangement(s) of radiation sources emitting ultraviolet radiation directed onto a human body in the intended position;
- devices for positioning the human body to be irradiated in a UV radiation-receiving relationship to the radiation sources;
- one or more devices for safeguarding the spectral distribution of ultraviolet radiation emitted by UV radiation sources, according to claims 1 to 17; and
- devices for electrical or electronic control of the one or more arrangement(s) of radiation sources emitting ultraviolet radiation in the direction of the human body as a function of the data acquired in the one or more device(s) according to claims 1 to 17.

19. An irradiator according to claim 18, being a tanning apparatus for tanning in the recumbent, standing or sitting position, preferably being a tanning apparatus for tanning in the recumbent position, more preferably being a whole body tanning apparatus for tanning in the recumbent position.

20. An irradiator according to claim 18 or claim 19 in the form of a tanning apparatus for tanning in the recumbent position, comprising
- a stationary lower part with head end and foot end and two longitudinal sides, containing electrical units, switching devices and connecting devices; and
- an upper part with head end and foot end and two longitudinal sides, either fitted to one of the longitudinal sides of the lower part via hinges or joints, tiltable or swivellable by means of the hinges or joints away from the lower part and towards the lower part to form a tanning chamber or liftable away from the lower part and lowerable towards the lower part to form a tanning chamber by means of a separate suspension; wherein
- the lower part comprises a lying surface for the user which is permeable at least to tanning UV radiation and at least one set of lamps arranged beneath the lying surface in the longitudinal direction of the tanning apparatus and optionally also further lamps for outputting radiation with at least a proportion of tanning UV radiation towards the upper part and/or at least a part of the user's body, in direct or indirect connection with the electrical units, switching devices and/or connecting devices; and
- the upper part comprises a protective surface for the user which is permeable at least to tanning UV radiation and at least one set of lamps arranged above the protective surface in the longitudinal direction of the tanning apparatus and optionally also further lamps for outputting radiation with at least a proportion of tanning UV radiation towards the lower part and/or at least a part of the user's body; and comprising
- one or more devices for safeguarding the spectral distribution of ultraviolet radiation emitted by UV radiation sources, according to claims 1 to 17.
